Europäisches Patentamt

European Patent Office

Office européen des brevets

(11) Veröffentlichungsnummer: **0 527 867 B1**

# (12) EUROPÄISCHE PATENTSCHRIFT

(45) Veröffentlichungstag der Patentschrift: **21.09.94**

(51) Int. Cl.5: **C08F 220/04**, A61L 15/00

(21) Anmeldenummer: **91909230.4**

(22) Anmeldetag: **10.05.91**

(86) Internationale Anmeldenummer:
**PCT/EP91/00878**

(87) Internationale Veröffentlichungsnummer:
**WO 91/18031 (28.11.91 91/27)**

(54) **VERNETZTES, WASSERABSORBIERENDES POLYMER UND VERWENDUNG ZUR HERSTELLUNG VON HYGIENEARTIKELN.**

(30) Priorität: **11.05.90 DE 4015085**

(43) Veröffentlichungstag der Anmeldung:
**24.02.93 Patentblatt 93/08**

(45) Bekanntmachung des Hinweises auf die
Patenterteilung:
**21.09.94 Patentblatt 94/38**

(84) Benannte Vertragsstaaten:
**AT BE CH DE DK ES FR GB GR IT LI LU NL SE**

(56) Entgegenhaltungen:
**US-A- 4 286 082**

**Houben-Weyl: 'Methoden der Organischen Chemie', Volume 14/1, page 265**

(73) Patentinhaber: **Chemische Fabrik Stockhausen GmbH**
**Bäkerpfad 25**
**D-47805 Krefeld (DE)**

(72) Erfinder: **MERTENS, Richard**
**Dahlerdyk 116 a**
**D-4150 Krefeld (DE)**
Erfinder: **DAHMEN, Kurt**
**Von-Velsen-Strasse 6**
**D-4050 Mönchengladbach (DE)**
Erfinder: **BREHM, Helmut**
**Dachsstrasse 22**
**D-4150 Krefeld (DE)**

(74) Vertreter: **Klöpsch, Gerald, Dr.-Ing. Patentanwalt**
**An Gross St. Martin 6**
**D-50667 Köln (DE)**

EP 0 527 867 B1

**Beschreibung**

Die Erfindung betrifft ein vernetztes, wäßrige Flüssigkeit absorbierendes Polymer mit verbesserten Eigenschaften hinsichtlich Quellung und Rückhaltevermögen von wässrigen Flüssigkeiten unter Belastung, sowie mit einem sehr geringen Restmonomergehalt und hoher Gelfestigkeit in gequollenem Zustand und die Verwendung dieser Polymeren zur Herstellung von Hygieneartikeln.

Die Einsatzmöglichkeiten für diese wasserabsorbierenden Polymere sind weit gestreut, wie z.B. in Babywindeln, in Artikeln für die Erwachseneninkontinenz, in Kabelummantelungen, in Wischtüchern oder auch zur Bodenverbesserung.

Ihre hauptsächliche Verwendung finden diese Polymere bei der Absorption von körpereigenen Flüssigkeiten, hier besonders Urin, und damit in Babywindeln und in der Erwachseneninkontinenz. Für diese Anwendung haben sich bisher als besonders geeignet Stärke-/Acrylsäure-Pfropfpolymerisate und vernetzte Acrylsäure-Salz-Polymere erwiesen, wie sie aus den Patentschriften US 4,076,663 bzw. EP 0036463 bekannt sind.

Während in der Vergangenheit wasserabsorbierende Polymere mit sehr hohem Quellvermögen bei Kontakt mit Flüssigkeit, auch freie Quellkapazität genannt, zur Windelherstellung bevorzugt wurden, werden heute Polymere mit einem völlig veränderten Eigenschaftsbild gefordert.

Nachdem erkannt worden ist, daß die wasserabsorbierenden Polymere in der Windel mechanisch belastet werden, verursacht durch die Bewegungen des Windelträgers, werden an die Polymere neue Anforderungen, nämlich hohe Retention, d.h. hohes Rückhaltevermögen für wäßrige Flüssigkeit und hohe Aufnahme für wäßrige Flüssigkeit unter Druckbelastung, gestellt. Weiterhin sollen die Polymere aus toxikologischen Gründen einen geringen Restmonomergehalt und einen niedrigen Gehalt an extrahierbaren Anteilen aufweisen.

Da sowohl die Vernetzerkonzentration und die Vernetzerart als auch die Polymerisationskatalysatoren diese vier Eigenschaften in wasserabsorbierenden Polymeren sehr stark beeinflussen, ist die Herstellung eines Polymers, das eine hohe Retention, eine hohe Aufnahme unter Belastung, einen geringen Restmonomergehalt und niedrige extrahierbare Anteile haben soll, mit großen Schwierigkeiten verbunden.

Während eine hohe Retention nur bei einem schwach vernetzten Polymer erreicht wird, erfordert eine hohe Aufnahme unter Belastung ein stark vernetztes, stabiles Polymer, das erst aufgrund dieser Stabilität gegen den äußeren Druck anquellen kann. Mit zunehmender Vernetzung steigt aber gleichzeitig auch der Restmonomergehalt an, weil durch die schnell ansteigende Viskosität des Polymergels während seiner Herstellung sich die Diffusion der restlichen Monomermoleküle zu den reaktiven Radikalzentren verringert. Restmonomerabsenkende Katalysatorsysteme oder erhöhte Katalysatorkonzentration verursachen oft eine unerwünschte Abnahme der Retention oder einen Anstieg der extrahierbaren Anteile. Die extrahierbaren Anteile eines vernetzten Polymers nehmen mit steigender Vernetzerkonzentration ab.

In der EP 0083022 werden wasserabsorbierende Polymere beschrieben, die eine hohe freie Quellkapazität aufweisen und nicht zum Verklumpen neigen. Die Herstellung dieser Polymere erfolgt durch Vernetzung der Polymerpartikel mit Glycidylethern.

Die US-Schrift 4,535,098 beschreibt wasserabsorbierende Polymere mit Anteilen an hydrophoben Comonomeren, die eine verbesserte Gelstabilität zeigen.

Die EP 0205674 lehrt die Herstellung von absorbierenden Polymeren durch Polymerisation der Acrylsäure in ihrer Säureform und Einstellung des Neutralisationsgrades nach beendeter Polymerisation. Durch diese Verfahrensweise werden Polymere mit verringerten löslichen Anteilen und einer verbesserten Gelstabilität erhalten. In Abhängigkeit vom Vernetzungsgrad liegt die Retention zwischen 20 und 74 g 0,9%ige NaCl-Lösung pro 1 g Polymer. Nachteilig ist die lange Polymerisationszeit und die aufwendige Neutralisation des Polymergels.

In den EP's 0287970 und 0251314 wird eine UV- bzw. Elektronenbestrahlung beschrieben, um geringe Restmonomergehalte in wasserabsorbierenden Polymeren zu erreichen.

Die DE-OS 3738602 beschreibt quellbare, wasserabsorbierende Pfropfpolymerisate, die durch radikalische Polymerisation von bevorzugt Acrylsäure erhalten werden. Die Patentschrift lehrt jedoch weder die Verwendung von Formamidinsulfinsäure zur Herstellung von verbesserten wasserabsorbierenden Polymeren noch macht sie irgendwelche Angaben über die Retention, die Aufnahme unter Druck oder den Restmonomergehalt der beschriebenen Pfropfpolymerisate.

In der DP 975794 wird die Herstellung von Kationenaustauschern aus unlöslichen Polymerisaten der (Meth)acrylsäure oder ihrer Salze unter Verwendung des trimeren Anlagerungsproduktes von Formaldehyd an Acrylnitril als Vernetzer beschrieben. Bei dem trimeren Anlagerungsprodukt handelt es sich um N,N', N''-Trisacryloyl-hexahydrotriazin. Die Patentschrift lehrt, daß die Polymerisation mit Redox-Systemen durchgeführt werden kann, die aus Persulfaten und Sulfoxy-Reduktionsmitteln bestehen; u.a. wird Formamidinsulfinsäure genannt. Es werden hochvernetzte Polymere erhalten mit geringen Retentionswerten. Über Umsatz bzw. Restmonomergehalte werden keine Angaben gemacht.

Die geforderte Eigenschaftskombination von hoher Retention und hoher Aufnahme unter Druck und geringem Restmonomergehalt und niedrige extrahierbare Anteile wird von den bekannten wasserabsorbierenden Polymeren nicht erreicht.

Aufgabe der Erfindung ist daher die Schaffung eines wasserabsorbierenden Polymeren, das hohe Retention und hohe Aufnahme von wäßrigen Flüssigkeiten unter Druck sowie geringen Restmonomergehalt und niedrige extrahierbare Anteile in sich vereinigt.

Diese Aufgabe wird erfindungsgemäß durch ein vernetztes, wässrige Flüssigkeiten oder Wasser absorbierendes, pulverförmiges Polymer gelöst, das erhalten wird durch Polymerisation einer Mischung aus

a) 60,0 bis 99,9 Gew. % ungesättigten, polymerisierbaren Monomeren mit Säuregruppen, die mindestens zu 30 Mol % neutralisiert vorliegen,

b) 0 bis 37 Gew. % wasserlöslichen, mit a) copolymerisierbaren Monomeren,

c) 0,1 bis 3,0 Gew. % Vernetzer und

d) 0 bis 10 Gew. % von wasserlöslichen Polymeren,

mit Wasser als wäßrige Lösung, Zerkleinerung, Trocknung und Mahlen des gebildeten Polymers, dadurch gekennzeichnet, daß als Vernetzer nach c) ein mehrfach ungesättigtes Monomer mit mindestens einer allylisch ungesättigten Gruppe oder Mischungen aus Polyvinyl- und Polyallylverbindungen im Gewichtsverhältnis 1 : ≥ 0,6 verwendet werden und daß die Polymerisation unter Verwendung eines Redoxsystems, das aus Formamidinsulfinsäure und einem oder mehreren organischen Peroxiden besteht, durchgeführt wird.

Diese Polymeren weisen eine Retention von größer gleich 28 g 0,9%iger wäßriger NaCl-Lösung pro 1 g Polymer, eine Aufnahme von größer gleich 26 g 0,9%iger wäßriger NaCl-Lösung pro 1 g Polymer bei einer Belastung mit 20 g/cm$^2$ und einen Restmonomergehalt kleiner als 700 ppm, bevorzugt kleiner als 500 ppm, und einen Gehalt an extrahierbaren Anteilen kleiner 6 % auf.

Die Verwendung von Formamidinsulfinsäure in Kombination mit Peroxiden zur Polymerisationsinitiierung ist bekannt (siehe: Houben-Weyl, "Methoden der organischen Chemie", Band 14/1, Seite 265); überraschend hat sich aber gezeigt, daß bei der Polymerisation in Gegenwart des Redox-Katalysatorsystems Formamidinsulfinsäure/Peroxid ein sehr hoher Umsatz der Monomeren zu Polymeren erreicht wird und der Restmonomergehalt im Polymeren < 700 ppm beträgt. Weiterhin war nicht vorhersehbar, daß die Retention der wasserabsorbierenden Polymere nicht verschlechtert, sondern - im Gegenteil - positiv beeinflußt wird und daß die Polymeren ebenfalls eine hohe Aufnahme unter Belastung zeigen.

Das wasserabsorbierende Polymer besteht zu 60 - 99,9 Gew.-% aus ungesättigten, polymerisierbaren Monomeren mit Säuregruppen, wie z.B. Acrylsäure, Methacrylsäure, 2-Acrylamido-2-methylpropansulfonsäure oder Mischungen dieser Monomeren; die Säuregruppen liegen mindestens zu 30 Mol.-% neutralisiert vor, so z.B. als Natrium-, Kalium- oder Ammoniumsalz.

Als weitere Monomere können die wasserabsorbierenden Polymere 0 - 37 Gew.-% Acrylamid, Methacrylamid, Hydroxyethylacrylat, Dimethylaminopropylacrylat, Dimethylaminopropylacrylamid oder Acrylamidopropyltrimethylammoniumchlorid enthalten. Höhere Anteile als 37 % dieser Monomerer verschlechtern die Quellfähigkeit der Polymeren.

Die mehrfach ungesättigten Monomere mit mindesten einer allylisch ungesättigten Gruppe nach Anspruch 1 sind ausgewählt aus den Verbindungsklassen

a) Allylester, z.B. Triallylcitrat, Diallylmaleinat, Allyl(meth)acrylat,

b) Polyallyläther, z.B. Glycerintriallyläther, Polyalkylenglykoläther des Allylglycidäther,

c) Allylamide, z.B. N,N'-Diallylbernsteinsäurediamid, N,N,N',N'-Tetraallyloxalsäurediamid, N-Allylacrylamid, N-Diallyl(meth)acrylamid oder

d) Polyallylamine wie z.B. Triallylamin, Tetraallylammoniumchlorid, Methyltriallylammoniumchlorid, N,N'-Tetraallylethylendiamin, N,N'-Tetraallylbutandiamin.

Als wasserlösliche Polymere können im wasserabsorbierenden Polymer 0 bis 10 Gew.-% teil- oder vollverseifter Polyvinylalkohol, Polyvinylpyrrolidon, Stärke oder Stärkederivate enthalten sein. Das Molekulargewicht dieser Polymeren ist unkritisch, solange sie wasserlöslich sind.

Die Polymerisation wird als Lösungspolymerisation entweder batchweise oder kontinuierlich durchgeführt. Dazu werden die Monomeren und der Vernetzer in Wasser gelöst, so daß die Monomerkonzentration der polymerisierenden Mischung 20 - 35 % beträgt, und durch Zugabe einer Base wird der gewünschte Neutralisationsgrad eingestellt, der mindestens 30 Mol.-% betragen soll. Ggf. können der Monomerlösung auch Netzmittel zugesetzt werden.

Weiterhin kann die Polymerisation im Dispersionszustand als inverse Suspension- oder Emulsionspolymerisation durchgeführt werden.

3

Die Polymerisation wird erfindungsgemäß mit einem Redox-Katalysatorsystem durchgeführt, das Formamidinsulfinsäure als reduzierende Komponente enthält und organische Peroxide als Oxidationskomponente. Cumolhydroperoxid und tertiäres Butylhydroperoxid sind wegen ihrer Wasserlöslichkeit bevorzugt.

Es ist ebenfalls möglich, daß die Formamidinsulfinsäure "in situ" aus Thioharnstoff und Wasserstoffperoxid gebildet wird. Unter "in situ" ist zu verstehen, daß anstelle von Formamidinsulfinsäure Thioharnstoff und Wasserstoffperoxid zur Monomerlösung gemischt werden und daß der Thioharnstoff in der Monomerlösung zur Formamidinsulfinsäure oxidiert wird.

Die Menge des Redox-Katalysators bewegt sich im üblichen Rahmen und liegt bei 100 - 2000 ppm Formamidinsulfinsäure, bezogen auf Monomerlösung, und bei 100 - 4000 ppm für das Peroxid.

Neben Formamidinsulfinsäure kann ein zweites Reduktionsmittel mitverwendet werden bis zu 10 Gew.-%, bezogen auf Formamidinsulfinsäure, z.B. Ascorbinsäure, Sulfit, Dihydroxymaleinsäure oder Hydroxylamin.

Als weitere Bestandteile der Polymerisationskatalyse können auch Azoverbindungen wie Azobisamidinopropan-Hydrochlorid eingesetzt werden.

Die Auslösung der Polymerisation kann durch die Formamidinsulfinsäure selbst bzw. ihre Alkalisalze oder mittels eines Co-Reduktionsmittels oder durch UV-Licht erfolgen.

Nach der Polymerisation wird das Polymergel zerkleinert, getrocknet und auf die gewünschte Partikelgröße abgesiebt.

Zur Charakterisierung der wasserabsorbierenden Polymere wird das pulverförmige Polymer auf 100-850 $\mu$m abgesiebt und diese Fraktion ggf. mit 0,1 bis 1,0 %, bevorzugt 0,5 % Aerosil 200, einer pyrogenen Kieselsäure, vermischt; anschließend werden Retention, Aufnahme unter Druck und Restmonomergehalt und extrahierbaren Anteil gemessen.

Die Retention wird nach der Teebeutelmethode bestimmt und als Mittelwert aus drei Messungen angegeben: ca. 200 mg Polymer werden in einen Teebeutel eingeschweißt und für 10 Minuten in 0,9%ige wäßrige NaCl-Lösung getaucht. Anschließend wird der Teebeutel in einer Schleuder bei 1.400 Upm 5 Minuten geschleudert und gewogen. Einen Teebeutel ohne wasserabsorbierendes Polymer läßt man als Blindwert mitlaufen:

$$\text{Retention} = \frac{\text{Auswaage} - \text{Blindwert}}{\text{Einwaage}} \quad (g/g)$$

Die Aufnahme unter Druck wurde mit einer Demand-Absorbency-Test-Apparatur bestimmt (siehe: "Allgemeiner Fließstoff-Report" 5/82, Seite 210-218), bei der der Prüftisch durch einen Büchner-Trichter ersetzt worden ist. Der Büchner-Trichter hat einen Durchmesser von 6,0 cm und wird so justiert, daß der Flüssigkeitsspiegel auf Höhe der Keramikfilterplatte liegt. Ein plangeschliffener Zylinder mit einem Durchmesser von 5,9 cm wird als Stempel benutzt, dessen Gewicht einen Druck von 20 g/cm$^2$ ausübt.

Zur Durchführung der Messung wird ein Rundfilterpapier in den Büchner-Trichter gelegt und 0,85 g wasserabsorbierendes Polymer der Kornfraktion 300-600 $\mu$m gleichmäßig aufgestreut. Anschließend wird der Zylinder eingesetzt und die Flüssigkeitsabsperrung geöffnet. Als Prüfflüssigkeit wird 0,9%ige wässrige NaCl-Lösung eingesetzt. Nach einer Stunde wird die Flüssigkeitsaufnahme als Verbrauch in der Bürette abgelesen.

Der Restmonomergehalt in den wasserabsorbierenden Polymeren wurde mittels HPLC bestimmt. Zur Bestimmung des löslichen Anteils wird das wasserabsorbierende Harz 1 Std. in 0,9 %iger NaCl-Lösung gerührt, anschließend wird filtriert und im Filtrat der Gehalt an Säuregruppen titriert.

Beispiel 1:

Eine wäßrige Acrylsäurelösung, die 0,5 % Diallylacrylamid, bezogen auf Acrylsäure, enthält, wurde unter Kühlung mit Natronlauge neutralisiert. Die Acrylsäurekonzentration der Monomerlösung betrug 30 % und der Neutralisationsgrad 70 %. 850 g der Monomerlösung wurden auf 10°C gekühlt und 10 Minuten mit Stickstoff gespült; anschließend wurden 3 g tertiäres Butylhydroperoxid, 4,5 mg Ascorbinsäure, gelöst in 10 g Wasser, und 500 mg Formamidinsulfinsäure, gelöst in 20 g Wasser, zugemischt. Die Polymerisation sprang sofort an, erkennbar an einer Temperaturerhöhung der Monomerlösung. Nach 30 Minuten wurde der entstandene Polymergelblock zerkleinert und mit Heißluft bei 150°C getrocknet.

Anschließend wurde das Polymer gemahlen, auf 100-850 $\mu$m abgesiebt und mit 0,5 % Aerosil 200 vermischt. Das wasserabsorbierende Polymer zeigte folgende Eigenschaften:

4

| Retention: | 29 g/g |
|---|---|
| Aufnahme unter Druck: | 27 g/g |
| Restmonomergehalt: | 255 ppm |
| Extrahierbarer Anteil: | 4,9 % |

Beispiel 2:

Zu 850 g Monomerlösung, entsprechend Beispiel 1, mit der Änderung, daß 0,55 % Diallylacrylamid eingesetzt wurden, wurden 2,5 g tertiäres Butylhydroperoxid und 500 mg Formamidinsulfinsäure, gelöst in 30 g Pufferlösung von pH 8, gemischt. Die Polymerisation setzte sofort ein. Nach 30 Minuten wurde der entstandene Polymergelblock zerkleinert und mit Heißluft bei 150°C getrocknet.

Anschließend wurde das Polymer gemahlen, auf 100-850 $\mu$m abgesiebt und mit 0,5 % Aerosil 200 vermischt. Das wasserabsorbierende Polymer zeigte folgende Eigenschaften:

| Retention: | 29 g/g |
|---|---|
| Aufnahme unter Druck: | 26 g/g |
| Restmonomergehalt: | 390 ppm |
| Extrahierbarer Anteil: | 4,4 % |

# Beispiel 3:

850 g Monomerlösung, entsprechend Beispiel 2, wurden mit 2,5 g tertiärem Butylhydroperoxid und 400 mg Formamidinsulfinsäure, die zu 10 Mol.-% mit Natronlauge neutralisiert in 30 g Wasser gelöst war, vermischt. Die Polymerisation setzte sofort ein. Der entstandene Polymergelblock wurde, wie in Beispiel 2 beschrieben, zu einem wasserabsorbierenden Polymer aufgearbeitet.

| Retention: | 31,5 g/g |
|---|---|
| Aufnahme unter Druck: | 27,0 g/g |
| Restmonomergehalt: | 370 ppm |
| Extrahierbarer Anteil: | 5,0 % |

Beispiel 4:

Eine wäßrige Acrylsäurelösung, die 0,9 % Triallylamin, bezogen auf Acrylsäure, enthält, wurde unter Kühlung zu 70 Mol.-% mit Kalilauge neutralisiert; die Acrylsäurekonzentration betrug 28 %.

850 g der Monomerlösung wurden auf 10°C gekühlt und 10 Minuten mit Stickstoff gespült; anschließend wurden 3 g tertiäres Butylhadroperoxid, 500 mg Formamidinsulfinsäure, gelöst in 20 g Wasser, und 4,5 mg Ascorbinsäure, gelöst in 10 g Wasser, zugemischt. Die Polymerisation sprang sofort an. Nach 30 Minuten wurde der entstandene Polymergelblock - wie in Beispiel 1 beschrieben - zu einem wasserabsorbierenden Polymer aufgearbeitet.

| Retention: | 29,5 g/g |
|---|---|
| Aufnahme unter Druck: | 27,0 g/g |
| Restmonomergehalt: | 290 |
| Extrahierbarer Anteil: | 4,5 % |

Vergleichsbeispiele 5 - 8:

Tabelle I zeigt die Eigenschaften von vier wasserabsorbierenden Polymeren, die von den Firmen Norsolor, Dow Chemical, Allied Colloids und Chemische Fabrik Stockhausen vermarktet werden. Sie stellen vernetzte, teilneutralisierte Polyacrylate dar.

Tabelle I

|  | Retention g/g | Aufnahme unter Druck g/g | Restmonomergehalt ppm |
|---|---|---|---|
| Norsocryl B | 32 | 18 | 130 |
| Drytech 510 | 33 | 12 | 560 |
| Salsorb 90 P | 28 | 21 | 500 |
| Favor SAB 922 | 39 | 9 | 350 |

Beispiele 9 - 12:

Es wurden wäßrige Acrylsäurelösungen mit unterschiedlichen Mengen Natronlauge neutralisiert und entsprechend Beispiel 1 polymerisiert. Tabelle II zeigt die Polymerisationsbedingungen und die Eigenschaften der wasserabsorbierenden Polymere.

Tabelle II

| Bei-spiel | Acrylsäure-konzentrat. | Neutralisa-tionsgrad | TAA | Ascorbin-säure | t.-BHP |
|---|---|---|---|---|---|
| 9 | 25 % | 70 % | 0,8 % | 2 mg | 3,0 g |
| 10 | 30 % | 50 % | 0,9 % | 4 mg | 2,5 g |
| 11 | 28 % | 90 % | 0,8 % | 9 mg | 2,0 g |
| 12 | 30 % | 70 % | 1,0 % | 4 mg | 2,5 g [1) |

Tabelle II (Fortsetzung):

| Bei-spiel | FAS | Retention | Aufnahme unter Druck | Restmonomer-gehalt | Extrah. Anteil |
|---|---|---|---|---|---|
| 9 | 250 mg | 32 g/g | 29 g/g | 380 ppm | 5,5 % |
| 10 | 500 mg | 31 g/g | 27 g/g | 220 ppm | 4,7 % |
| 11 | 500 mg | 28 g/g | 26 g/g | 430 ppm | 4,7 % |
| 12 | 500 mg | 30 g/g | 29 g/g | 330 ppm | 3,9 % |

[1): In Beispiel 12 wurden 2,5 g Cumolhydroperoxid anstelle von tertiärem Butylhydroperoxid eingesetzt.

```
TAA               = Triallylamin
tertiäres BHP = tertiäres Butylhydroperoxid
FAS               = Formamidinsulfinsäure
```

Beispiele 13 - 17:

Acrylsäure (AS) wurde in wäßriger Lösung mit Methacrylsäure (MAS), Acrylamid (AA), 2-Acrylamido-2-methylpropansulfonsäure (AMPS) bzw. Dimethylaminopropylacrylamid (DIMAPA) copolymerisiert in Gegenwart von 1 % Triallylamin. Die Monomerkonzentration betrug 30 % und der Neutralisationsgrad 70 %. Als Katalysatorsystem wurden 9 mg Ascorbinsäure, 3 g tertiäres Butylhydroperoxid und 500 mg Formamidinsulfinsäure verwendet; Tabelle III zeigt die Copolymerzusammensetzung und die Eigenschaften der wasserabsorbierenden Polymere:

## Tabelle III

| Bei-spiel | AS Gew.-% | Comonomer Gew. % | Retention |
|-----------|-----------|------------------|-----------|
| 13 | 90 | 10 AMPS | 31,5 g/g |
| 14 | 65 | 35 AMPS | 30,5 g/g |
| 15 | 75 | 25 AA | 30,0 g/g |
| 16 | 70 | 30 MAS | 29,0 g/g |
| 17 | 90 | 10 DIMAPA | 31,0 g/g |

## <u>Tabelle III (Fortsetzung):</u>

| Bei-<br>spiel | Aufnahme<br>unter Druck | Restmonomer-<br>gehalt | Extrahierb.<br>Anteil |
|------|------|------|------|
| 13 | 29,5 g/g | 380 ppm | 3,5 % |
| 14 | 28,5 g/g | 290 ppm | 2,0 % |
| 15 | 28,0 g/g | 370 ppm | 2,2 % |
| 16 | 28,0 g/g | 220 ppm | 4,8 % |
| 17 | 28,0 g/g | 240 ppm | 5,2 % |

<u>Vergleichsbeispiele 18 - 23:</u>

850 Monomerlösung mit 30 % Acrylsäure, die zu 70 Mol.-% neutralisiert vorliegt, wurde mit verschiedenen Katalysatoren initiiert; in Tabelle IV sind die Katalysatoren und die Eigenschaften der erhaltenen wasserabsorbierenden Polymere aufgeführt:

Tabelle IV

| Bei-spiel | TAA % | Ascorbin-säure | t.BHP | NPS | $H_2O_2$ | ABAH |
|-----------|-------|----------------|-------|-----|----------|------|
| 18 | 1,0 | 9 mg | 3 g | | | |
| 19 | 1,0 | 9 mg | 3 g | | | 1 g |
| 20 | 1,0 | 9 mg | 3 g | 0,6 g | | |
| 21 | 1,0 | 9 mg | | 0,6 g | 50 mg | |
| 22 | 0,9 | | 2 g | | | |
| 23 | 0,8 | | 2 g | | | |

NPS = Natriumperoxidisulfat

Tabelle IV (Fortsetzung):

| Bei-spiel | Dithionit | Formaldehyd-sulfoxylat | Retention |
|-----------|-----------|------------------------|-----------|
| 18 | | | 27 g/g |
| 19 | | | 29 g/g |
| 20 | | | 25 g/g |
| 21 | | | 25 g/g |
| 22 | 0,5 g | | 30 g/g |
| 23 | | 100 mg | 31 g/g |

## <u>Tabelle IV (Fortsetzung):</u>

| Bei-spiel | Aufnahme unter Druck | Restmonomergehalt | Extrahierb. Anteil |
|-----------|----------------------|-------------------|--------------------|
| 18 | 22 g/g | 3.380 ppm | 4,1 % |
| 19 | 29 g/g | 2.430 ppm | 5,1 % |
| 20 | 27 g/g | 1.960 ppm | 5,6 % |
| 21 | 27 g/g | 1.490 ppm | 5,6 % |
| 22 | 26 g/g | 6.200 ppm | 6,2 % |
| 23 | 22 g/g | 9.110 ppm | 4,9 % |

<u>Beispiele 24 - 32:</u>

Zu 850 g Monomerlösung mit 30 % Acrylsäure, die zu 70 Mol.-% mit Natronlauge neutralisiert worden war, wurden verschiedene Vernetzer zugesetzt. Anschließend wurde die Polymerisation - wie in Beispiel 1 beschrieben - mit 4,5 mg Ascorbinsäure gelöst, in 10 g Wasser, mit 3 g tertiärem Butylhydroperoxid und 500 mg Formamidinsulfinsäure, gelöst in 20 g Wasser, initiiert. Die Eigenschaften der erhaltenen wasserabsorbierenden Polymere sind in Tabelle V zusammengestellt:

Tabelle V
===

| Beispiel | Vernetzer | Retention |
|---|---|---|
| 24 Vergleich | 0,8 % TMPTA | 30 g/g |
| 25 Vergleich | 1,2 % TMPTA | 29 g/g |
| 26 | 0,5 % TMPTA / 0,4 % TAA | 28 g/g |
| 27 | 0,5 % TMPTA/ 0,8 % TAC | 29 g/g |
| 28 Vergleich[1] | 11,1 % N-Trisacryloly-hexahydro-triazin | 9 g/g |
| 29 Vergleich[2] | 0,3 % N-Trisacryloyl-hexahydro-triazin | 24 g/g |
| 30 | 0,25 % MBA/ 0,25 % TAA | 28 g/g |
| 31 | 1,0 % N,N'-Tetraallylbutandiamin | 31 g/g |
| 32 | 0,5 % N-Allylacrylamid | 28 g/g |

Tabelle V (Fortsetzung):

| Beispiel | Aufnahme unter Druck | Restmonomer- gehalt | Extrahierb. Anteil |
|---|---|---|---|
| 24 Vergleich | 17 g/g | 210 ppm | 12,3 % |
| 25 Vergleich | 18 g/g | 340 ppm | 11,0 % |
| 26 | 26 g/g | 405 ppm | 5,8 % |
| 27 | 27 g/g | 470 ppm | 5,6 % |
| 28 Vergleich[1] | 10 g/g | 8,1 % | 0,8 % |
| 29 Vergleich[2] | 24 g/g | 6,9 % | 2,2 % |
| 30 | 26 g/g | 290 ppm | 5,8 % |
| 31 | 28,5 g/g | 390 ppm | 4,3 % |
| 32 | 28 g/g | 230 ppm | 3,8 % |

TMPTA = Trimethylolpropantriacrylat

TAA = Triallylamin

TAC = Triallylcitrat

MBA = Methylenbisacrylamid

[1]: Beispiel 28 wurde entsprechend Beispie 1 der DP 975794

**hergestellt.**

**2: Beispiel 29 wurde mit 0,35 g Formamidinsulfinsäure/ 0,5 g Persulfat/ 9mg Ascorbinsäure hergestellt.**

Beispiel 33:

850 ml Monomerlösung aus Beispiel 1 mit 1 % Triallylamin anstelle von Diallylacrylamid, die zusätzlich 6,7 g gelösten Polyvinylalkohol enthielt, wurde - wie in Beispiel 1 beschrieben - polymerisiert und zu einem wasserabsorbierenden Polymer aufgearbeitet, das 2 % Polyvinylalkohol enthält.

| Retention: | 30 g/g |
|---|---|
| Aufnahme unter Druck: | 29 g/g |
| Restmonomergehalt: | 370 g/g |
| Extrahierbarer Anteil: | 3,9 % |

Beispiel 34:

Eine wäßrige Acrylsäurelösung, die 0,7 % Triallylamin enthält, wurde unter Kühlung mit Natronlauge neutralisiert, und anschließend wurde eine wäßrige Stärkelösung (Typ: Sobex 222 der Firma Südstärke GmbH) zugemischt. Die Acrylsäurekonzentration betrug 26,5 % und der Neutralisationsgrad 70 Mol.-%. 935 g dieser Monomerlösung wurden auf 10°C gekühlt, 10 Minuten mit Stickstoff gespült und anschließend mit 3 g tertiärem Butylhydroperoxid, 500 mg Formamidinsulfinsäure, gelöst in 20 g Wasser, und 4,5 mg Ascorbinsäure, gelöst in 10 g Wasser, vermischt. Die Polymerisation setzte sofort ein. Nach 30 Minuten wurde der erhaltene Gelblock zerkleinert und mit Heißluft bei 110°C getrocknet. Das getrocknete Polymer enthält 8,5 % Stärke.

| Retention: | 30,5 g/g |
|---|---|
| Aufnahme unter Druck: | 27,5 g/g |
| Restmonomergehalt: | 310 ppm |
| Extrahierbarer Anteil: | 5,2 % |

Beispiel 35

850 g Monomerlösung aus Beispiel 1 mit 1 % Triallylamin anstelle von Diallylacrylamid wurden auf 10°C gekühlt und 10 Minuten mit Stickstoff gespült. Anschließend wurden in folgender Reihenfolge 0,35 g Thioharnstoff, gelöst in 20 g Wasser, 1,0 g Wasserstoffperoxid, 3 g tertiäres Butylhydroperoxid und 5 mg Ascorbinsäure, gelöst in 10 g Wasser, zur Monomerlösung gemischt, worauf die Polymerisation sofort ansprang. Nach 30 Minuten wurde der entstandene Polymergelblock zerkleinert und mit Heißluft bei 150°C getrocknet.

| Retention: | 28 g/g |
|---|---|
| Aufnahme unter Druck: | 28 g/g |
| Restmonomergehalt: | 400 ppm |
| Extrahierbarer Anteil: | 4,3 % |

Die Verwendung der erfindungsgemäßen, wasserabsorbierenden Polymere wurde in schichtartig aufgebauten Konstruktionen aus Fluff und wasserabsorbierendem Polymer geprüft. Runde Konstruktionen aus 3 Fluff-Schichten und 2 Schichten wasserabsorbierendem Polymer (Durchmesser 5,8 cm) wurden in den Büchner-Trichter gelegt, mit dem die Aufnahme unter Druck bestimmt wird. Bei einer Druckbelastung von 20 g/cm$^2$ läßt man die Konstruktionen 150 Minuten lang 0,9%ige NaCl-Lösung saugen, wonach die Aufnahme des wasserabsorbierenden Polymers wie folgt berechnet wird.

$$\textbf{Aufnahme} = \frac{(\textbf{Verbrauch in der Bürette}) - \textbf{Blindwert für den Fluff})}{\textbf{Einwaage}}$$

Tabelle VI

| Anwendungsbeispiele 36 - 41: | | | |
|---|---|---|---|
| Beispiel | Polymer aus Beispiel | Anteil Polymer (1) in der Konstruktion | Aufnahme (ml/g) |
| 36 | 1 | 20 | 35 |
| 37 | 1 | 40 | 31 |
| 38 | 12 | 20 | 36 |
| 39 | 12 | 40 | 32 |
| 40 | 33 | 20 | 35 |
| 41 | 33 | 40 | 31 |
| Vergleichsbeispiel | | | |
| FAVOR SAB 922 (2) | | 20 | 29 |
|  | | 40 | 25 |
| FAVOR SAB 954 (2) | | 20 | 29 |
|  | | 40 | 27 |

(1) Gew.-%, bezogen auf Fluff
(2) Handelsprodukte der Firma Chemische Fabrik Stockhausen GmbH

Bevorzugt werden die erfindungsgemäßen Polymeren zur Herstellung von Körperflüssigkeiten absorbierenden Konstruktionen eingesetzt, die für den Einsatz in Windein- und Hygieneartikeln geeignet sind und die aus 98 - 30 Gew.% hydrophilen Fasern und 2 - 70 % wasserabsorbierenden Polymeren bestehen.

**Patentansprüche**

1. Vernetztes, wäßrige Flüssigkeiten oder Wasser absorbierendes, pulverförmiges Polymer, erhältlich durch Polymerisation einer Mischung aus

a) 60,0 bis 99,9 Gew. % ungesättigten, Polymerisierbaren Monomeren mit Säuregruppen, die mindestens zu 30 Mol % neutralisiert vorliegen,
b) 0 bis 37 Gew. % wasserlöslichen, mit a) copolymerisierbaren Monomeren,
c) 0,1 bis 3,0 Gew. % Vernetzer und
d) 0 bis 10 Gew. % von wasserlöslichen Polymeren,

mit Wasser als wäßrige Lösung, Zerkleinerung, Trocknung und Mahlen des gebildeten Polymers, dadurch gekennzeichnet, daß als Vernetzer nach c) ein mehrfach ungesättigtes Monomer mit mindestens einer allylisch ungesättigten Gruppe oder Mischungen aus Polyvinyl- und Polyallylverbindungen im Gewichtsverhältnis 1 : ≧ 0,6 verwendet werden und daß die Polymerisation unter Verwendung eines Redoxsystems, das aus Formamidinsulfinsäure und einem oder mehreren organischen Peroxiden besteht, durchgeführt wird.

2. Vernetztes, wässrige Flüssigkeiten oder Wasser absorbierendes Polymer nach Anspruch 1, gekennzeichnet durch eine Retention von größer gleich 28 g 0,9 %iger wäßriger NaCl-Lösung pro 1 g Polymer, eine Aufnahme von größer gleich 26 g 0,9 %iger wäßriger Na-Cl-Lösung pro 1 g Polymer bei einer Belastung mit 20 g/cm$^2$, einen Restmonomergehalt kleiner als 700 ppm, bevorzugt kleiner als 500 ppm, und einen Gehalt an extrahierbaren Anteilen kleiner 6 %.

3. Vernetztes, wässrige Flüssigkeiten oder Wasser absorbierendes Polymer nach Anspruch 1 und 2, wobei das Redox-Katalysatorsystem neben Formamidinsulfinsäure weitere Reduktionsmittel enthalten kann.

4. Vernetztes, wässrige Flüssigkeiten oder Wasser absorbierendes Polymer nach einem der Ansprüche 1 bis 3, wobei die Formamidinsulfinsäure "in situ" aus Thioharnstoff und Wasserstoffperoxid gebildet wird.

5. Vernetztes, wässrige Flüssigkeiten oder Wasser absorbierendes Polymer noch einem der Ansprüche 1 bis 4, wobei als Säuregruppen-tragende Monomere Acrylsäure, Methacrylsäure und 2-Acrylamido-2-methylpropansulfonsäure oder Mischungen dieser Monomeren eingesetzt werden.

6. Vernetztes, wässrige Flüssigkeiten oder Wasser absorbierendes Polymer nach einem der Ansprüche 1 bis 4, wobei als wasserlösliche Polymere Polyvinylalkohol, Polyvinylpyrrolidon, Stärke oder Stärkederivate verwendet werden.

7. Verwendung des vernetzten, wässrige Flüssigkeiten oder Wasser absorbierenden Polymers nach Ansprüchen 1 bis 6 zur Herstellung von Hygieneartikeln.

8. Verwendung des vernetzten, wässrige Flüssigkeiten oder Wasser absorbierenden Polymers nach Anspruch 6 zur Herstellung von Körperflüssigkeiten absorbierenden Konstruktionen, die für den Einsatz in Windeln und Hygieneartikeln geeignet sind, bestehend aus 98 - 30 Gew. % hydrophiler Fasern und 2 - 70 % wasserabsorbierendem Polymer.

## Claims

1. A cross-linked, powdery polymer absorbing aqueous liquids or water, obtainable by polymerizing a mixture consisting of
   a) 60.0 to 99.9%-wt. unsaturated, polymerizable monomers with acid groups which are neutralized to the extent of at least 30 mol-%,
   b) 0 to 37%-wt. water-soluble monomers copolymerizable with a),
   c) 0.1 to 3.0%-wt. of a cross-linking agent, and
   d) 0 to 10%-wt. of water-soluble polymers,
   with water as aqueous solution, crumbling, drying, and grinding the formed polymer,
   characterized in that a multiply unsaturated monomer with at least one allylically unsaturated group or mixtures of polyvinyl and polyallyl compositions at a weight ratio of 1 : $\geq$ 0.6 is used as cross-linking agent according to c), and that the polymerization is carried out with a redox system consisting of formamidine sulfinic acid and one or more organic peroxides.

2. The cross-linked polymer absorbing aqueous liquids or water according to claim 1, characterized by a retention of greater than or equal to 28 g 0.9% aqueous NaCl-solution per 1 g of polymar, an absorption of greater than or equal to 26 g 0.9% aqueous NaCl-solution per 1 g of polymer under a load of 20 g/cm$^2$, a residual monomer content of less than 700 ppm, preferably less than 500 ppm, and a content of extractable portions of less than 6%-wt.

3. The cross-linked polymer absorbing aqueous liquids or water according to claims 1 and 2, wherein the redox catalyst system may comprise further reducing agents in addition to formamindine sulfinic acid.

4. The cross-linked polymer absorbing aqueous liquids or water according to any one of claims 1 to 3, wherein the formamidine sulfinic acid is formed "in situ" from thiourea and hydrogen peroxide.

5. The cross-linked polymer absorbing aqueous liquids or water according to any one of claims 1 to 4, wherein acrylic acid, methacrylic acid and 2-acrylamido-2-methylpropane sulfonic acid or mixtures of said monomers are used as acid-groups-containing monomers.

6. The cross-linked polymer absorbing aqueous liquids or water according to any one of claims 1 to 4, wherein polyvinyl alcohol, polyvinylpyrrolidone, starch or starch derivatives are used as water-soluble polymers.

7. The use of the cross-linked, aqueous liquids or water absorbing polymer as defined in claims 1 to 6 in the production of hygiene articles.

8. The use of the cross-linked, aqueous liquids or water absorbing polymer according to claim 6 in the production of constructions which are capable of absorbing body liquids and are suitable for the use in diapers and hygiene articles, consisting of 98 to 30%-wt. of hydrophilic fibers and 2 to 70% of a water-absorbing polymer.

**Revendications**

1. Polymère réticulé, pulvérulent, absorbant les liquides aqueux ou l'eau, que l'on peut obtenir par la polymérisation d'un mélange constitué de
   a) 60,0 à 99,9% en poids de monomères insaturés, polymérisables, comportant des radicaux acide, qui sont neutralisés jusqu'à au moins 30% molaires,
   b) 0 à 37% en poids de monomères solubles dans l'eau, copolymérisables avec a),
   c) 0,1 à 3,0% en poids d'agents de réticulation et
   d) 0 à 10% en poids de polymères solubles dans l'eau,
   avec de l'eau, sous forme de solution aqueuse, le broyage, le séchage et la mouture du polymère formé, caractérisé en ce qu'à titre d'agents de réticulation selon c), on utilise un monomère polyinsaturé comportant au moins un radical à insaturation allylique, ou des mélanges de composés polyvinyliques et polyallyliques, dans le rapport pondéral 1:7, 0,6 et en ce que l'on entreprend la polymérisation en recourant à l'emploi d'un système redox, constitué d'acide formamidinesulfinique et d'un ou plusieurs peroxydes organiques.

2. Polymère réticulé, absorbant les liquides aqueux ou l'eau selon la revendication 1, caractérisé par une rétention égale ou supérieure à 28 g de solution aqueuse à 0,9% de NaCl pour 1 g de polymère, et d'une prise égale ou supérieure à 26 g de solution aqueuse à 0,9% de NaCl pour 1 g de polymère, sous une charge de 20 g/cm$^2$, une teneur résiduelle en monomère inférieur à 700 ppm, de préférence inférieure à 500 ppm et une teneur en fractions extractibles inférieure à 6% en poids.

3. Polymère réticulé, absorbant les liquides aqueux ou l'eau suivant les revendications 1 et 2, caractérisé en ce que le système de catalyseur redox peut contenir d'autres agents de réduction en plus de l'acide formamidinesulfinique.

4. Polymère réticulé, absorbant les liquides aqueux ou l'eau suivant l'une quelconque des revendications 1 à 3, caractérisé en ce que l'on forme l'acide formamidinesulfinique "in situ" à partir de thiourée et de peroxyde d'hydrogène.

5. Polymère réticulé, absorbant les liquides aqueux ou l'eau suivant l'une quelconque des revendications 1 à 4, caractérisé en ce que, à titre de monomères portant des radicaux acide, on utilise l'acide acrylique, l'acide méthacrylique et l'acide 2-acrylamido-2-méthylpropanesulfonique, ou des mélanges de ces monomères.

6. Polymère réticulé, absorbant les liquides aqueux ou l'eau suivant l'une quelconque des revendications 1 à 4, caractérisé en ce que l'on utilise le poly(alcool vinylique), la polyvinylpyrrolidone, l'amidon ou des dérivés d'amidon, à titre de polymères solubles solubles dans l'eau.

7. Utilisation du polymère réticulé, absorbant les liquides aqueux ou l'eau suivant l'une quelconque des revendications 1 à 6, en vue de la fabrication d'articles pour l'hygiène.

8. Utilisation du polymère réticulé, absorbant les liquides aqueux ou l'eau suivant la revendication 6 pour la fabrication d'ensembles absorbant les liquides corporels, qui conviennent pour la confection d'articles pour l'hygiène et de couches, qui se composent de 98 à 30% en poids de fibres hydrophiles et de 2 à 70% en poids de polymère absorbant l'eau.